# EUROPEAN PATENT APPLICATION

(11) **EP 0 823 480 A1**
(43) Date of publication of application: **11.02.1998**
(21) Application number: 96112684.4
(22) Date of filing: 06.08.1996
(51) Int. Cl.: C12N 15/82, A01H 5/00, C12N 15/62

(54) **Controlled gene expression in plants**

(71) Applicant: Max-Planck-Gesellschaft zur Förderung der Wissenschaften e.V., 80539 München (DE)
(72) Inventor: Palme, Klaus, Dr., D-50259 Pulheim (DE); Moore, Ian, Dr., GB-Oxford 0X4 3NN (GB); Gälweiler, Leo, D-55595 St. Katharinen (DE); Schell, Jozef, Prof. Dr., D-50829 Köln (DE); Grosskopf-Kroiler, Deborah, Dr., D-50859 Köln (DE)
(74) Representative: Maiwald, Walter, Dr. Dipl.-Chem.

(57) **Abstract**

This invention relates to the control of gene expression in plants using either a chimeric transcriptional activator protein system, or a system based on suppressor tRNAs. The chimeric transactivating proteins of the invention offer a variety of advantages, including the specific controlled activation of expression of genes engineered to comprise transactivator-responsive elements, thereby achieving exceptionally high levels of gene expression. In particular, the transactivator proteins of the invention comprise a DNA-binding portion of the prokaryotic Lac repressor operatively linked to a polypeptide which directly or indirectly activates transcription in eukaryotic cells. Further, the transactivation system comprises DNA molecules containing one or more transactivator-responsive promoter elements within a predetermined gene of interest in a second target DNA molecule. Alternatively, control of gene expression by transactivation can be achieved by coexpression of a suppressor tRNA gene and target gene containing a premature stop codon in a plant cell. Furthermore, the invention relates to transgenic plants carrying a DNA molecule coding the transactivator protein or a DNA molecule comprising the gene of interest combined with Lac repressor-responsive elements, crossing of a transgenic plant carrying a gene of interest under control of a Lac repressor/transactivator-sensitive promoter element with a transgenic plant coding for a Lac repressor/transactivator protein and transactivation of expression of a gene of interest. Analogously, the invention relates to transgenic plants carrying a DNA molecule coding a suppressor tRNA or a DNA molecule comprising a target gene which contains a premature stop codon, preferably an amber stop codon, crossing of a transgenic plant carrying a target gene containing a premature stop codon with a transgenic plant coding for the corresponding suppressor tRNA and transactivation of a target gene.

## Description

This invention relates to the control of gene expression in plants using either a chimeric transcriptional activator protein system, or a system based on suppressor tRNAs. The chimeric transactivating proteins of the invention offer a variety of advantages, including the specific controlled activation of expression of genes engineered to comprise transactivator-responsive elements, thereby achieving exceptionally high levels of gene expression. In particular, the transactivator proteins of the invention comprise a DNA-binding portion of the prokaryotic Lac repressor operatively linked to a polypeptide which directly or indirectly activates transcription in eukaryotic cells. Further, the transactivation system comprises DNA molecules containing one or more transactivator-responsive promoter elements within a predetermined gene of interest in a second target DNA molecule. Alternatively, control of gene expression by transactivation can be achieved by coexpression of a suppressor tRNA gene and target gene containing a premature stop codon in a plant cell. Furthermore, the invention relates to transgenic plants carrying a DNA molecule coding the transactivator protein or a DNA molecule comprising the gene of interest combined with Lac repressor-responsive elements, crossing of a transgenic plant carrying a gene of interest under control of a Lac repressor/transactivator-sensitive promoter element with a transgenic plant coding for a Lac repressor/transactivator protein and transactivation of expression of a gene of interest. Analogously, the invention relates to transgenic plants carrying a DNA molecule coding a suppressor tRNA or a DNA molecule comprising a target gene which contains a premature stop codon, preferably an amber stop codon, crossing of a transgenic plant carrying a target gene containing a premature stop codon with a transgenic plant coding for the corresponding suppressor tRNA and transactivation of a target gene.

One important aim of reverse genetics is to study and resolve the biological functions of isolated gene material in organisms. Also, the ability to identify genes that control many aspects of plant growth and function is now central to much plant science. Both basic and applied research relies increasingly on modification of specific genes to manipulate biological processes in transgenic plants. Thus antisense RNA, dominant negative mutants, ectopic expression, and over-expression technology have provided powerful tools to investigate a broad range of biochemical pathways. However, these molecular genetic approaches can not always be easily applied to the genes that control fundamental processes of plant growth and development. This is primarily because manipulating such critical genes can be severely detrimental to plant growth and survival and so preclude the generation and propagation of useful transgenic plants for analysis and industrial applications.

In the case of plant genes, functional study is realised by introduction and expression of these genes in receptor plants. The transferred genetic material can be stably incorporated into the receptor genome and may cause modifications upon its over-expression or ectopic expression. Such phenotypic characteristics and modifications in transgenic plants may provide important information about the function of the respective gene and its gene product. With respect to the analysis of such transgenic plants it is highly desirable that phenotypic abnormalities, which are observed in comparison to wild type plants, can be traced back unambiguously to the ectopic expression of the transferred, foreign gene material.

Production of stable transgenic plants can be carried out by several different techniques. In principle, foreign DNA is introduced into a plant cell, incorporated in the plant genome and finally an intact, whole plant develops from a single transformed plant cell. Very often, the last step involves regeneration of whole plants from totipotent, undifferentiated plant cells, used as transformation material.

It is known that the analysis of transgenic plants and the interpretation of observed phenotypes can be dramatically hindered by difficulties arising during the whole process of plant cell transformation and subsequent regeneration of transgenic plants. Unfortunately, very often it is impossible to relate the characteristics of the emerged phenotypes exclusively to the expression of the introduced foreign genes. Genetic and epigenetic modifications, which emerge during regeneration and which are called somaclonal variations, may lead to abnormal phenotypes not unambigously related to the expression of the introduced foreign genes. For example, the incorporation of foreign DNA into the plant genome may result in deletions, which themselves lead to phenotypic modifications. Moreover, the incorporation of foreign DNA into regions of the plant genome may cause totally independent modifications, e.g. by randomly knocking out or modifying genes having no connection with the introduced DNA information.

Another difficulty emerges from the expression of foreign genes coding for proteins, which themselves - or the products of the reactions catalyzed by these proteins - have a specific influence on the transformed plant cells. Thus, in case the expression of a foreign gene has a detrimental or even toxic effect on the regeneration and production of whole transgenic plants, it is extremely likely that only such plants are selected during regeneration, which express the gene of interest either not at all or at a very low level. Consequently, the resulting phenotypes may lead to misinterpretations of the function of the gene of interest. Very often, for the above mentioned reasons, transgenic plants expressing a foreign gene at a desired level cannot be produced at all and it is difficult to determine whether this results from trivial errors in experimental design or from the biological activity of the transgene.

One solution to the problem has been to use "tissue-specific" promoters to restrict the activity of transgenes to certain tissues. This approach is far from satisfactory as many such promoters are active during the process of regeneration of transgenic plants and also active at a low level in many tissues. It also restricts the scope of the analysis to a few cell types and does not always allow the appropriate tissues to be studied. Another option is the use of heat-shock promoters which can achieve relatively high level expression in many cell types. This has the disadvantage that the subsequent analysis is performed on plants subjected to heat stress, growth conditions must be carefully controlled, and problems have been encountered with expression of genes in non-heat induced plants. To overcome these limitations several attempts have been made to develop chemically inducible gene expression systems. These are again not entirely satisfactory as they are either relatively inefficient, dependend on sustained gene repression, limited to single plant species, or reliant on the application of chemicals at concentrations that may be toxic to the plants. A very well-studied system is a tetracycline inducible promoter system developed for tobacco (Gatz et al. (1991) Mol. Gen. Genet. 227, 229-237; Gatz et al. (1992) Plant J. 2, 397-404). In this case a modified 35S RNA promoter from Cauliflower Mosaic Virus is repressed in plants that express high levels of the Escherichia coli Tetracycline (Tet)-repressor, but full activity is recovered when tetracycline is applied at 0.1 to 1.0 µg/ml. Such chemical induction systems offer useful temporal control of gene expression in tobacco cells though their usefulness and reliability for expression of genes in various tissues of whole plants is less clear.

Thus, there is a need for a system for effective spatial control of transgene expression in plants that does not require external intervention or imposition of environmental stress.

Further, there is a need for a system that makes quantitatively controlled gene expression in plants possible and by the use of which the correlation between a specific phenotype and the expression and the level of expression of introduced genes can be unambiguously demonstrated.

Furthermore, since the process of gene construction and transgenic plant regeneration is time consuming and labour intensive, there is a need to simplify procedures for generating a series of transgenic plants in each of which the gene of interest is expressed under a different temporal, spatial or physiological regime as required.

Other approaches for controlled gene expression in eukaryotic cells based on transcription activator systems have been reported.

WO91/19784, for example, discloses a chimeric transcriptional activator protein system functioning in transfected mammalian cells. The transactivating proteins comprise a functional portion of a DNA-binding protein and a portion of a vertebrate or viral transcriptional activator protein. The DNA binding protein may be the bacterial Lac repressor and the transcriptional activator protein used may be VP16 of Herpes simplex virus. The ability to control the gene expression and to switch the transactivator function on and off by use of the described system is only mentioned in the context of transgenic animals and mammalian cell lines.

Similarly, EP 0 455 424 relates to the development of a novel mammalian inducible and cascading gene expression system. Specifically the invention relates to the development of stable mammalian host cell lines, coexpressing the HIV TAT protein under the regulation of lacI/lacO and a cloned gene under transcriptional control of the HIV LTR promoter. Thus, this document describes an inducible gene expression system for controlled expression in mammalian cell lines.

WO94/29442 also relates to a regulatory system allowing for conditional inactivation or modulation of expression of a gene of interest in a host cell or animal. The system, based on a tetracycline-controllable transactivator (tTA) and a tTA-responsive promoter is described for use in transgenic animals.

Referring to transactivating systems functioning in plants, US Patent 5,362,864 discloses the isolation and characterisation of a plant transactivating factor, TAF-1. Further it is suggested to engineer TAF-1 into cell culture systems or transgenic plants to increase or modulate the expression of heterologous genes fused to promoter elements containing one or more copies of cis-acting sequences known to be bound by TAF-1.

Patent Application WO95/20668 describes a method for the production of modified plants, comprising crossing a first line with a second line to produce a plant having a phenotypic trait, wherein neither the first or second lines possess the phenotypic trait and wherein at least one of the parent plants is transgenic. In particular, the method is to be used for the production of male sterile plants and restoration of male sterility by expression of restorer genes. Thus, the application describes a method for the production of plants which are useful in hybrid seed production.

Similarly, Patent Application EP 0 589 841 relates to a method for producing male sterile plants based on an anther-specific promoter region operably linked to a sequence encoding a transactivator polypeptide and a target sequence capable of being activated by the transativator polypeptide operably linked to a sequence encoding antisense RNA or a polyphenol capable of disrupting viable pollen formation. This invention thus also relates to a method for producing male sterile plants and hybrid seed.

European Patent Application 0 494 724 describes plasmids for controlling expression in plants comprising DNA sequences that permit the expression, controlled in respect of time and location, of a heterologous product and plants. In principle, the control system relies on inducing expression of a gene of interest by inactivating a Tet repressor protein bound to its operator sequences within the respective promoter region via tetracycline.

It is known that the ability of transcriptional activators to bind to DNA and to simultaneously activate transcription is localised in defined domains of such transcription factors. It could be demonstrated by several experiments that transcriptional activator factors consist of independently functioning modules (Ptashne (1988) Nature 355, 683-689; Mitchell and Tijan (1989) Science 245, 371-378).

Thus, it is possible to combine a portion responsible for transcription activation of one factor with a DNA binding portion of another factor. The resulting hybrid protein is fully active in cells. This could be shown for the first time in yeast cells (Ptashne and Gann (1990) Nature 346, 329-331; Lewin (1990) Cell 61, 1161-1164; Saha et al. (1993) Nature 363, 648-652). Moreover, this observation could be confirmed for several transcription factors found in mammals (Guarante (1988) Cell 52, 303-305; Sadowski et al. (1988) Nature 335, 563-564).

However, the use of chimeric transcriptional activators, comprising a DNA binding portion functional in plants and a transcription activating portion functional in plants, for controlling transgene expression in plants is new.

It is worth noting that results obtained by expression of transcription activators in bacteria, yeast or mammalian cells can not be simply transferred to plant systems. For example the methylation pattern of the target DNA, differing in different cell types, makes binding of the transcription activator and thus gene activation impossible or at least very inefficient.

In bacteria, repressor proteins are used to reversibly bind to operator sequences controlling the expression of non-constitutive genes (as e.g. structure genes involved in metabolic pathways). Particularly, the regulation of the Lac operon of E. coli has been intensively studied for the last decades. Both, the Lex and Lac repressors have been used in mammalian cells (Brown et al (1987) Cell 49, 603-612; Ho and Davidson (1987) Cell 48, 555-566, Smith et al (1988) EMBO J. 7, 3975-3982). It was also shown that the Lac repressor can inhibit expression of eucaryotic viral promoters containing operator sequences downstream of the TATA-box, downstream from a vaccina virus promoter, or from a SV40 promoter in mammalian cells (Brown et al, supra; Ho and Davidson, supra).

Unfortunately, represssion is generally not complete and it is unclear if high levels of induced expression can be obtained in plants with these vectors.

While the Lac repressor has been observed to function in mammalian cells indicating that lacI can interact with DNA in the context of mammalian chromatin, advantageous use of the lacI DNA binding domain in plants is new, and furthermore, in the light of the foregoing, an unexpected result.

It has been found in prokaryotic cells that some lacI mutants, differing from wild type lacI in the DNA binding portion, have a superior binding capacity to the wild type DNA target, the Lac operator found in Escherichia coli, and to specifically modified Lac operator sequences, respectively (Lehming et al (1987) The EMBO Journal 6, 3145-3153).

Particularly, the use of lacI mutants and taking advantage of their superior binding capacity to the wild type or modified operator sequences have never been discussed in the context of transgene activation in plants.

Transcriptional control of the galactose and melibiose metabolic pathways in Saccharomyces cerevisiae is positively mediated through the regulatory protein GAL4. The presence of galactose GAL4 divergently promotes transcription of the genes of the galactose regulon. Transcriptional activation by GAL4 results in a 1,000 fold increase in the level of gene expression (Johnston (1987) Microbiol. Rev 51, 458-476). In the absence of galactose as inducer the negative regulatory protein GAL80 inhibits the transactivating ability of GAL4 in yeast.

GAL4 binds to a specific target DNA sequence, consisting of 17 base-pairs which exhibit dyad symmetry and which are termed the galactose upstream activating sequence.

Binding of GAL4 to its target DNA sequence is insufficient for directing RNA polymerase II-dependent transcription of linked genes. Rather, the DNA binding function of the protein serves the purpose to position the carboxyterminal transcription activating domains in the vicinity of the promoter and the transcription machinery. Transcriptional activation is mediated by two major activating domains termed activating region I (amino acid residues 148-196) and activating region II (amino acid residues 767-881), of which activating region II is the more potent (Ma and Ptashne (1987) Cell 48, 847-853).

Native GAL4 transcription activator has been demonstrated to activate transcription of genes linked to the GAL4 binding site in insect and mammalian cells (Kakidani and Ptashne (1988) Cell 52, 161-167; Webster et al (1988) Cell 52, 169-178). However, full length GAL4 is incapable of stimulating transcription in plant protoplasts, possibly as a result of its inefficient synthesis or instability (Ma et al (1988) Nature 334, 631-633).

Another very potent transcription activator is the Herpes Simplex Virus (HSV) virion protein 16 (VP16; Sadowski et al., supra; Triezenberg et al. (1988) Genes & Dev. 2, 718-729). So far, VP16 has been described only in the context of transactivation in mammalian cells (see preceding prior art discussion, WO91/19784).

Recently, transgene expression of a gene of interest arranged in trans with a control gene in Drosophila has been reported (Brand and Perrimon (1993) Development 118, 401-415; Crieg and Akam (1993) Nature 362, 630-632). However, neither of these reports discuss the possibility of using such a system in plants.

In another strategy F1 hybrids that express characteristics that would be undesirable in the parental inbred lines can be obtained by a suppressor tRNA transactivation system.

Expression of translation factors which permit or enhance translation of particular messenger RNAs is frequently used by microorganisms. For example, the use of suppressor transfer RNAs, i.e. tRNAs which are capable of reading stop codons, allows translation of an mRNA containing a premature termination codon. This phenomenon which has been extensively studied in bacteria has been reviewed by Eggertson and Söll (1988) Microbiol. Rev. 52, 354-374; Atkisnon and Martin (1994) Nucleic Acid Research 22, 1327-1334. This concept is also successfully realized in several plant viruses. For example, tobacco mosaic virus and tobacco rattle virus use natural weak suppressor tRNAs present in plant cells to read through leaky stop codons in their mRNAs (Beier et al. (1984) EMBO J. 3, 351-356; Zerfass and Beier (1992) Plant J. 2, 583-588; Carneiro et al. (1993) Plant Mol. Biol. 22, 681-690; Ulmasov and Folk (1995) Plant Cell 7, 1723-1734).

However, so far no biotechnological applications have been proposed. Thus, here for the first time tRNAs are described as useful for biotechnological purposes due to their central role in translation.

In general, there is a need for being able to turn gene expression "on" and "off", or regulating the level of gene expression in an efficient and controlled manner without causing pleiotropic effects, gene suppression, silencing or cytotoxicity.

However, so far no highly efficient trans-activating system for control of gene expression in plants has been developed. Therefore, there is a need for a superior control system based on novel chimeric transcription activators and responsive promoters. Further, there is need for totally new transactivation systems in order to broaden the spectrum of plant biotechnological tools, so that the person skilled in the art may choose the most efficient transactivation strategy depending on the plant to be transformed, the target gene, and/or other factors that may have an impact on breeding a desired plant.

Thus, one object of the invention is to provide a transcriptional activator protein, ensuring strong trans-activation of expression of a gene of interest in plants.

Chimeric transcriptional activators having strong DNA binding capacity as well as strong activation potential in plants offer an advantageous tool for adjusting expression of a gene of interest in plants. Further, the chimeric transcriptional activator protein should bind its DNA targets in a highly specific manner, thus limiting expression activation to the gene of interest which comprises one or more specific transcriptional activator-responsive elements.

Another object of the invention is to provide a new expression control system based on suppressor tRNA genes and target genes containing premature stop codons. Such a system should be relatively simple to handle in the plant to be transformed and highly efficient in the transactivation of a gene of interest, i.e. the background level of expression should be as low as possible.

Using the transcription activator system it may also be possible to manipulate the quantitiy of expression of the transgene (i) by modifying the level of transactivator expression and (ii) by adjusting the number of transactivator-responsive elements within the gene of interest. The same applies for the suppressor tRNA system by the use of which expression of the target gene can be adjusted by the expression level of the tRNA gene and/or the expression level of the target gene.

Another object of the invention is to enable the expression of genes, the gene product of which, or the chemical compound resulting from the reaction catalysed by the gene product, being cytotoxic or having any other detrimental or lethal effect. It is further an object of the invention to enable the expression of genes, the gene products of which being e.g. pump channels or pores, may be useful or detrimental e.g. as a consequence of moving one or more chemical compound, element or ion from one place to another.

Another object of the invention is to provide plants expressing the desired phenotypic trait only fully once the gene of interest and the genetic information coding for the chimeric transcriptional activator are co-expressed in the same plant cell. However, it is also sometimes desirable that expression of the desired phenotypic trait may be reversed, e. g. by separating apart of the regulatory and phenotype transgenes in subsequent plant generations.

It is also an object of the invention to provide plants expressing the desired phenotypic trait only fully once the gene of interest containing a premature stop codon and the suppressor tRNA gene are co-expressed in the same plant cell. Also here, it may be desired that the phenotypic trait is reversed, e. g. by separating apart of the regulatory and phenotype transgenes in subsequent plant generations.

Another object is to provide a control-system which may be used when safety implications have to be taken, i.e. for example when the release of a phenotypic trait into the environment is to be prevented.

Another object of the invention is to produce and isolate a gene product of interest, e.g. in large scale production in transgenic field trials.

Another object of the invention is to prevent problems of co-suppression experienced with conventional cis-acting systems.

These and other objects are attained by the subject matters defined in the attached independent claims. Preferred embodiments are notable from the dependent claims and the present description.

The invention provides a chimeric transcriptional activator which is active in plants comprising a functional portion of a DNA-binding protein and a functional portion of a transcriptional activator protein. In particular, the invention provides a chimeric transactivator protein comprising a functional portion of the lacI repressor protein of Escherichia coli.

In a preferred embodiment, the chimeric transactivator protein of the invention comprises as DNA binding portion a portion of a mutant lacI protein, having at least one amino acid exchange. Particularly, a lacI protein mutant having an exchange from tyrosine (amino acid 17) to histidine in position 1 of the DNA recognition helix of the lacI protein of Escherichia coli (referred to as H1-mutant). As will be further illustrated below, the H1-Lac repressor mutant provides exceptional binding capacity of the chimeric transcription activator protein of the invention to specific responsive elements.

A preferred transactivating protein used in this invention is GAL4 from Saccharomyces cerevisiae.

In particular, the invention provides a chimeric transactivator protein comprising a functional portion of the lacI repressor protein of Escherichia coli and a functional portion of the transcriptional activator protein GAL4 of S. cerevisiae.

Another preferred transcription activator protein used in this invention is VP16 of HSV.

In particular, the invention provides a chimeric transactivator protein comprising a functional portion of the lacI repressor protein from E. coli and a functional portion of the transcriptional activator protein VP16 of Herpes Simplex Virus.

Further this invention provides recombinant DNA molecules coding for the different chimeric transcriptional activator proteins of the invention.

The invention comprises transgenic plants, in the cells of which a gene of interest combined with Lac operator sequences is integrated into the plant genome.

In preferred embodiments of the present invention, lacI or a lacI mutant having one or more amino acid exchanges, particularly in their DNA recognition helix, are the bacterial DNA binding proteins used as a backbone for the chimeric transactivator protein and the transactivator-responsive element is the wild-type Lac operator sequence or a modified Lac operator sequence having one or more different base-pairs and/or a loss of at least one base-pair in comparison to the wild type Lac operator sequence, AATTGTGAGCGGATAACAATT or the wild type Lac operator consensus sequence, AATTGTGAGCSGCTCACAATT (S being G or C, see Lehming (1990) "Regeln für Protein/DNA-Erkennung", Dissertation, Universität zu Köln).

Use of Lac operator sequences as transactivator-responsive elements within the gene of interest is especially advantageous since such target sequences which allow for binding of the transactivator protein are most likely restricted to those sequences which are transferred to the plant. Thus, no undesired binding to homologous sequences will affect the trans-control system in plants.

The invention also provides recombinant DNA molecules containing either a suppressor tRNA gene or a target gene comprising a premature stop codon, preferably an amber stop codon. In a preferred embodiment one DNA molecule comprises either a modified tRNA^{Ser} or a tRNA^{Leu} gene under the control of a weak promoter, such as for instance the nopaline synthase (nos) promoter or the 1'/2' promoter from Agrobacterium tumefaciens (Depickter et al. (1982) J. Mol. Appl. Genet. 1, 561-573, and Velten et al. (1984) EMBO J. 12, 2723-2730, respectively) to allow expression of a strong amber suppression tRNA, but to avoid toxic or mutagenic effects by the ectopically expressed suppressor tRNA. The gene of interest, contained within the other DNA molecule can be any gene. Preferrably the target gene comprises a premature amber stop codon to interrupt protein translation resulting in production of a nonfunctional protein, unless the corresponding suppressor tRNA is coexpressed in the same plant cell. While the suppressor tRNA gene should be expressed under control of a rather weak promoter in order to avoid toxic effects of the suppressor tRNA, the target gene can be expressed under control of any promoter functional in plant cells. Preferrably, the gene of interest carrying a premature stop codon is expressed under a strong promoter, as e.g. the 35S RNA promoter of Cauliflower Mosaic Virus. This combination will provide very high activation of the expression levels of the target gene(s) of interest in the bhybrid F1 plant obtained after crossing of both transgenic plants carrying either the tRNA suppressor component or the target gene component of this binary transactivation system. The invention further provides the transgenic plants comprising these transgenes.

The plants that may be transformed with the recombinant nucleic acid molecules of the invention can be any plant. Dependent on the desired phenotypic trait, it may be gymnosperms, dicotyledons or monocotyledons.

The invention also includes plant reproduction products of the plants of the invention, for example seeds, fruits, cuttings, tubers, rhizomes, and parts of the plant, as protoplasts, plant cells, calli or roots.

The invention provides methods for controlling the expression of a gene of interest comprising utilisation of the chimeric transactivator proteins of the invention, thus allowing specific regulation of the expression of the gene of interest which comprises one or more transactivator-responsive elements.

The invention also provides methods for controlling the expression of a gene of interest comprising utilisation of suppressor tRNAs of the invention, thus specifically regulating expression levels of the gene of interest which comprises at least one premature stop codon.

Since the regulatory properties and the phenotypic properties of the transgenes, which come together in an F1 hybrid plant after crossing of the respective parent plants comprising either the gene of interest or expressing the transcriptional activator protein or the suppressor tRNA, respectively, will segregate apart in subsequent generations, the trans-control system of the invention may also be advantageously employed when it is desirable to reverse expression of the desired phenotypic trait.

Additionally, as binding of lacI to operator sequences in bacteria and mammals can be alleviated by use of galactosides and galactoside analogs (e.g. isopropyl-β-D-thiogalactoside (IPTG), methyl-β-D-thiogalactoside (MTG)) the application of such compounds to plants, plant cells or protoplasts may be used to repress binding of lacI to its target sequences, and thus to repress transactivation, if desired.

Further the invention comprises methods of producing plants having a desired phenotypic trait, the method comprising transforming a first plant with a recombinant nucleic acid molecule encoding a chimeric lacI (or lacI mutant)-GAL4- or lacI (or lacI mutant)-VP16-transcriptional activator protein, transforming a second plant with a recombinant nucleic acid molecule comprising the gene of interest as well as at least one lacI (or lacI mutant)-responsive element, crossing of the first and second plants and obtaining trans-activation when both transgenes are co-expressed in the same plant cell. It is self-evident, that any minimal promoter or other promoter systems that is inactive in the plant cell(s) of interest in the absence of the Lac-activator binding may be used in order to express the gene of interest by transactivation.

Further the invention comprises methods of producing plants having a desired phenotypic trait, the method comprising transforming a first plant with a recombinant nucleic acid molecule encoding a suppressor tRNA, transforming a second plant with a recombinant nucleic acid molecule comprising the gene of interest carrying at least one premature stop codon, preferably an amber stop codon, crossing of the first and second plants and obtaining trans-activation when both transgenes are coexpressed in the same plant cell.

Of course, transactivation of a gene of interest may also be achieved by directly transferring the nucleic acid molecule encoding the transactivator or the suppressor tRNA and the nucleic acid molecule encoding the gene of interest into the same plant, rather than by crossing. Thus, in some circumstances or for some applications it may be advantageous to introduce the two constructs in the same cells by other means such as serial transformation or infection with viral vectors. The presence of two independent nucleic acid molecules within the same plant or plant cell can be easily monitored using different selectable marker genes, or by standard techniques used in molecular biology.

Further the invention provides methods for producing plants which produce cytotoxic products.

Further the invention may be used for producing plants which produce a particular gene product in quantity.

The invention also provides a useful control system when safety implications have to be taken. For example, the chance of release of a phenotypic trait into the environment can be drastically reduced, e.g. when one of the transgenes is responsible for male sterility (no release of transgenic pollen, advantage for seed companies marketing transgenic seeds, reduction or complete avoidance of cosuppresion).

However, in general any gene of particular interest can be used in the approach of the invention.

The transactivator system of the invention may also be advantagously employed when it is desirable to use the same promoter to express several genes that must all be expressed in the same cell(s) to generate the desired phenotype, e.g. several enzymes in a biosynthetic pathway. The Lac-transactivator system involves the repeated insertion of only short sequences (the lac operator sequences) into the genome, reducing the risks of co-suppression.

It may also be desired to use a divergent promoter, i.e. two divergent minimal promoters each side of the Lac-operators which would be co-ordinately regulated by the Lac-activators. This may be of use if two genes of interest are to be expressed together, or if one gene is easily monitored (e.g. a reporter gene like the GUS gene) and the other is a gene of interest.

It is also possible by use of the transactivator system of the invention to generate a battery of transgenic plants that express the activator from a variety of different promoters. Thus it should be possible to express a gene of interest in many different patterns by simply crossing the battery of activator plants with transgenic plants containing the gene of interest under control of the lac-responsive element(s). This means that the gene of interest must be inserted into only one expression plasmid and used only once to generate a few transformants, rather than being recloned behind each promoter and plants being retransformed independently in each case. This will represent a considerable saving in time, labour and money.

In general, the transactivation systems of the invention show the advantages of being stably inherited, of being stable during the plant life cycle, of giving very high expression levels (e.g. similar to or even higher than those obtained by use of the strong viral 35S RNA promoter from CaMV in plants).

For the production of the new plants of the invention several different methods can be applied in accordance with this invention. On the one hand plants or plant cells can be modified by common transformation methods known in genetic engineering to that extent that the new DNA sequences are integrated in the plant genome, i. e. stable transformations are created. On the other hand also transient gene expression may be studied and employed.

The invention comprises a method for producing these new plants based on the recombinant nucleic acid molecules of the invention, their transfer to plant cells and their expression in plants. If desired, expression of the chimeric transcriptional activator protein can be controlled as well, for example by expressing this transgene under control of a tissue-specific, inducible, or developmentally controlled promoter region. It was already mentioned that the use of the transactivator system of the invention is particularly time-saving and labour-saving, when a gene of interest is to be expressed specifically in a variety of different cell types. Further, the level of transactivator expression can be analyzed in plants being hemizygous or homozygous for the transactivator coding DNA sequence. It is self-evident, that also the level of expression of the gene of interest may be varied due to the gene of interest being in the hemizygous or homozygous state, respectively. Transgenic lines which are homozygous for one or more new nucleic acid molecules can be easily identified by usual techniques, e.g. due to the segregation patterns of subsequent progenies. Further, the basic variation in expression (known as "position effects") found between independent transformants can also be used to vary the expression level of the gene of interest by its effect on both the activator and reporter T-DNA.

For introducing DNA into a plant host cell several techniques are available and the person skilled in the art can easily choose a suitable transformation procedure. These techniques comprise the transformation of plant cells with T-DNA using Agrobacterium tumefaciens or Agrobacterium rhizogenes as transformation means, fusion of protoplasts, direct gene transfer of isolated DNA into protoplasts, microinjection or electroporation of DNA, introducing DNA via biolistic methods and other procedures.

When the DNA is transferred by microinjection or electroporation any form of DNA may be used. The same applies for direct gene transfer. Here, simple plasmids, as for example pUC derivatives can be used. However, if whole plants are to be regenerated from transformed plant cells a marker gene which allows selection of transformed plants is necessary. Suitable selection markers are known to the person skilled in the art.

Depending on the transformation method used further DNA sequences may be required. For instance, if the Ti- or Ri-plasmid is used, the gene to be transferred has to be combined with at least the right border of the T-DNA contained in the Ti- and Ri-plasmid. Often, the gene to be transferred has to be combined with both the right and the left border of the T-DNA as flanking areas.

If agrobacteria are used for the transformation, the DNA to be transferred has to be cloned into specific plasmids, i.e. into an intermediary or a binary vector. Intermediary vectors comprise sequences homologous to sequences of the T-DNA which allow them to be integrated in the Ti- or Ri-plasmid of agrobacteria by homologous recombination. Further, this plasmid carries the vir-region which is required for the T-DNA transfer. Intermediary vectors are not capable of replicating in agrobacteria. The intermediary vector can be transferred to agrobacteria via a helper plasmid (conjugation). Binary vectors are able to replicate in both E. coli and agrobacteria. They contain a selection marker gene and a linker or polylinker framed by the left and right border regions of the T-DNA. These vectors can be directly transferred into agrobacteria (Holsters et al. (1978), Molecular and General Genetics 163, 181-187). The agrobacterial host cell should contain a plasmid carrying the vir-region which is necessary for the transfer of the T-DNA into the plant cell.

The transformed agrobacterium is used for the transformation of plant cells. The use of T-DNA for the transformation of plant cells is well-studied and suffiently described in EP 120 515; Hoekema in: The Binary Plant Vector System, Offsetdrokkerij Kanters B.V., Alblasserdam (1985) Chapter V; Fraley et al. (1993), Crit. Rev. Plant Sci. 4, 1-46 and An et al. (1985), EMBO J. 4, 277-287.

For the transfer of DNA into plant cells plant explants can be effectively co-cultivated with Agrobacterium tumefaciens or Agrobacterium rhizogenes. Subsequently, whole plants can be regenerated from the infected plant material (e.g. leaves or leaf pieces, stem segments, roots, protoplasts or plant cells cultivated in suspension cultures) in a suitable medium containing antibiotica or biocides as selection agents. The regeneration of the plants is accomplished by common regeneration methods using known nutrient media. The obtained plants can then be analyzed for the DNA that was to be transferred using standard biochemical and genetic engineering methods. Biolistic transformation methods can also be applied, as described e.g. in Willmitzer (1993) Transgenic Plants, in: Biotechnology, A Multi-Volume Comprehensive Treatise (H.J. Rehm, G. Reed, A. Pühler, P. Stadler, eds) Vol. 2, 627-659, V.C.H. Weinheim - New York - Basel - Cambridge).

While the transformation of dicotyledonous plants via A. tumefaciens using Ti-plasmid-vector systems is well established, recent studies seem to indicate that also monocotyledonous plants can be transformed via Agrobacterium-derived vectors (Chan et al. (1993), Plant Mol. Biol. 22, 491-506; Hiei et al. (1994), Plant J. 6, 271-282; Deng et al. (1990), Science in China 33, 28-34; Wilmink et al. (1992), Plant Cell Reports 11, 76-80; May et al. (1995) Bio/Technology 13, 486-492; Conner and Domiss (1992) Int. J. Plant Sci. 153, 550-555; Ritchie et al. (1993) Transgenic Res. 2, 252-265).

Alternative systems for the transformation of monocotyledons are procedures using the biolistic approach (Wan and Lemaux (1994), Plant Physiol. 104, 37-48; Vasil et al. (1993), Bio/Technology 11, 1553-1558; Ritala et al. (1994), Plant Mol. Biol. 24, 317-325; Spencer et al. (1990), Theor. Appl. Genet. 79, 625-631), transformation of protoplasts, electroporation of partially permeabilized cells, the introduction of DNA via glass fibres.

Transformation methods that can be used for the transformation of gymnosperms are described e.g. in Liu et al. (1993) Plant Mol. Biol. 23, 297-308; Primich and Minocha (1991) Plant Cell Reports 10, 545-549; Morris et al. (1989) Phys. Mol. Plant Pathol. 34, 451-462.

Once the introduced DNA is integrated into the plant genome, it remains stable and is also stably inherited to the progeny of the originally transformed plant cell. Usually, it contains a selection marker conferring resistance against a biocide or antibioticum as kanamycin, G418, bleomycin, hygromycin, methotrexate, glyphosate, streptomycin, sulphonyl urea, gentamycin, phosphinotricin, etc. The selection marker which can be chosen individually should therefore allow selection of transformed cells over cells which are devoid of the transferred DNA.

The transformed cells grow within the plant in the normal way (e.g. McCormick et al. (1986) Plant Cell Reports 5, 81-84). The resulting plants can be cultivated in the usual fashion and may be propagated by self-fertilization or be crossed with other plants, wild-type or transgenic plants. Transgenic plants containing reporter or activator (i.e. transactivator or tRNA encoding) constructs can be crossed and if the parents are heterozygous, progeny containing both activator and reporter T-DNAs can be identified by the presence of different resistance markers on each T-DNA. These different resistance markers also allow any phenotypes demonstrated by the progeny to be correlated with the presence or absence of each T-DNA. Further evidence linking phenotypes of progeny plants to expression of the gene of interest can be obtained by crossing activator and reporter plants respectively with reporter plants that lack lacI-responsive elements in the promoter of the target gene or with plants that express a mock activator lacking the transcription activation domain.

The following figures, tables and examples, describing the recombinant nucleic acid molecules, plants, as well as methods of the invention, are set forth for purposes of illustration only and are not to be construed as limitations on the present invention.

### FIGURES and TABLES

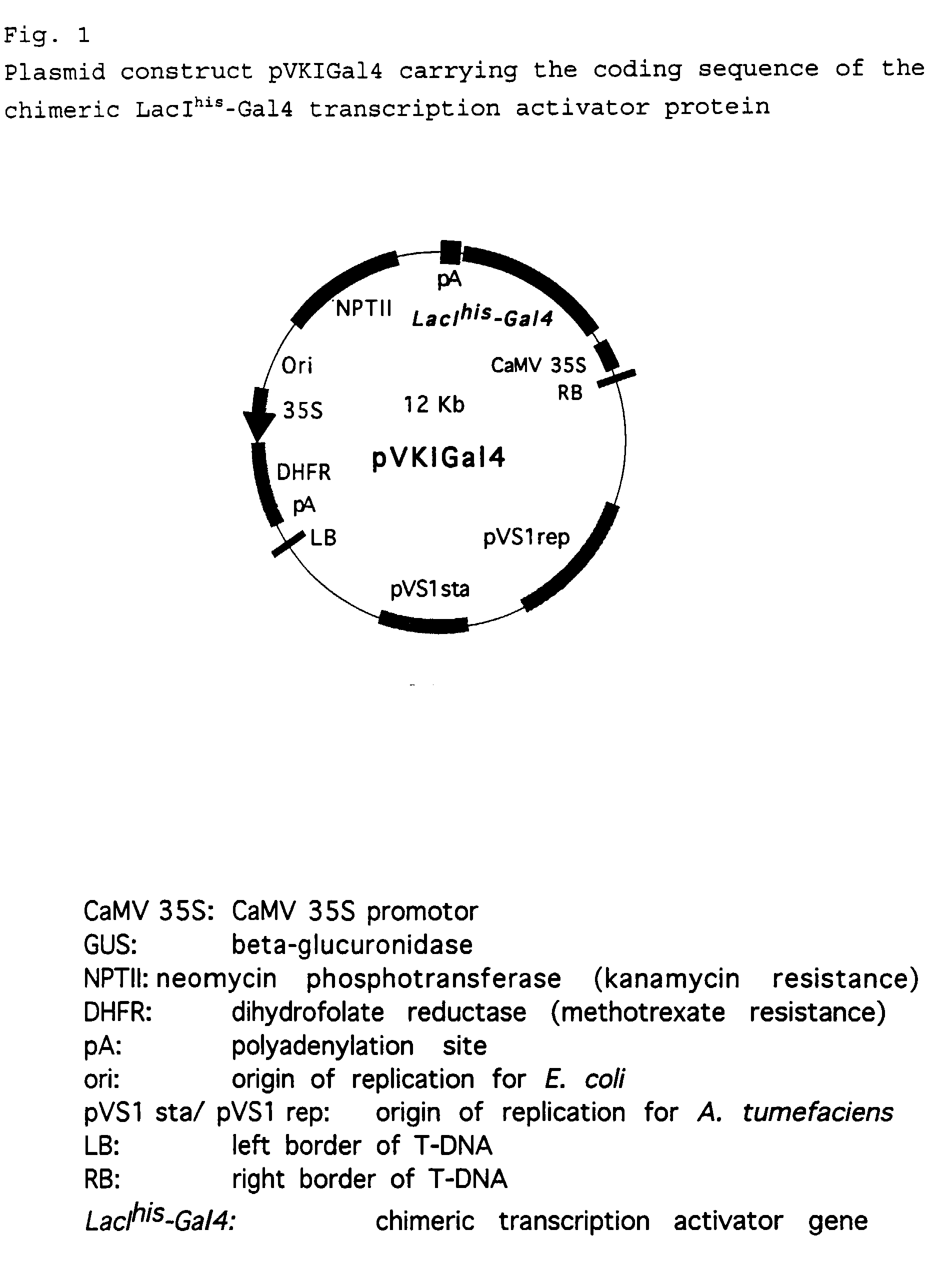

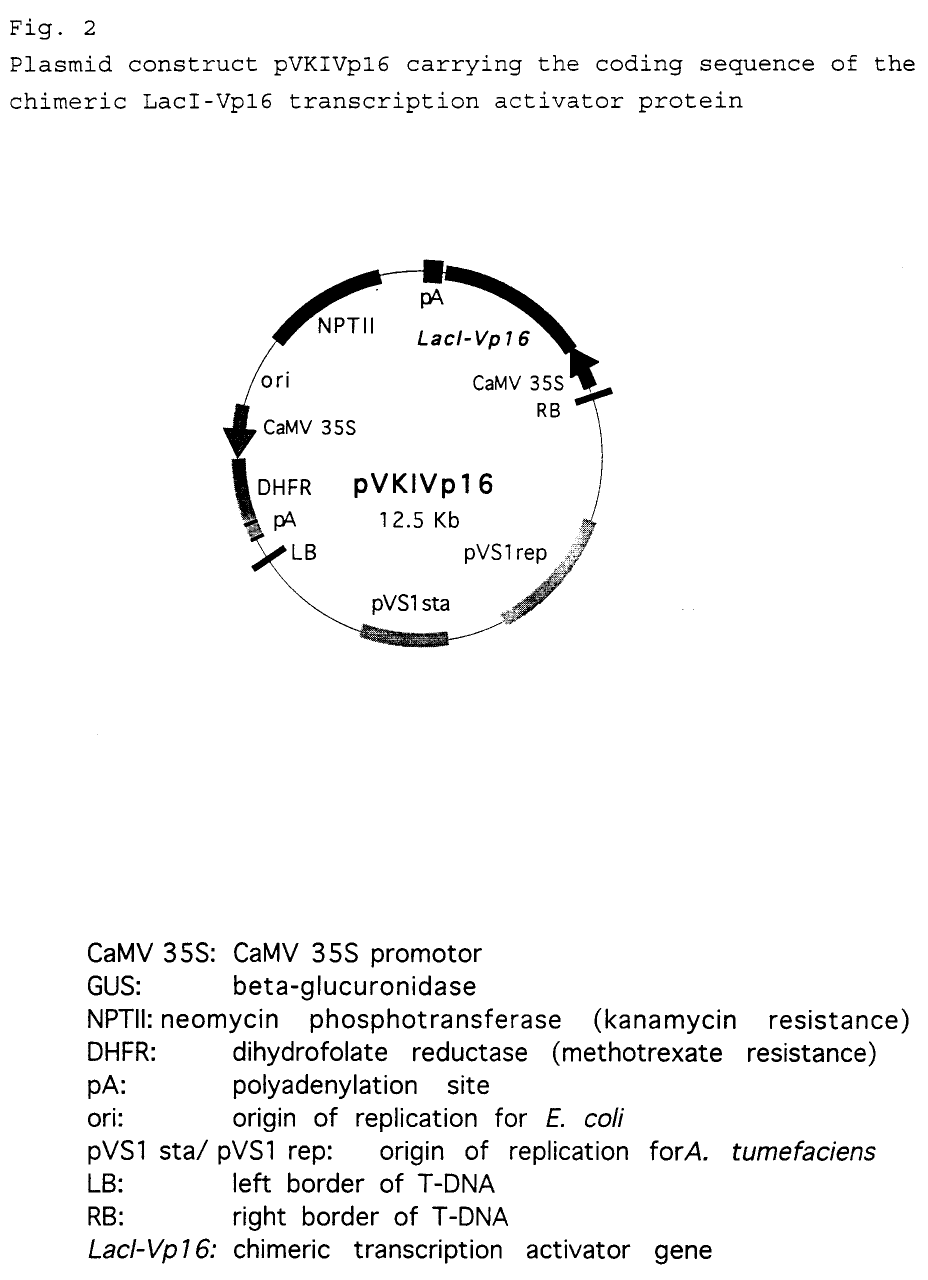

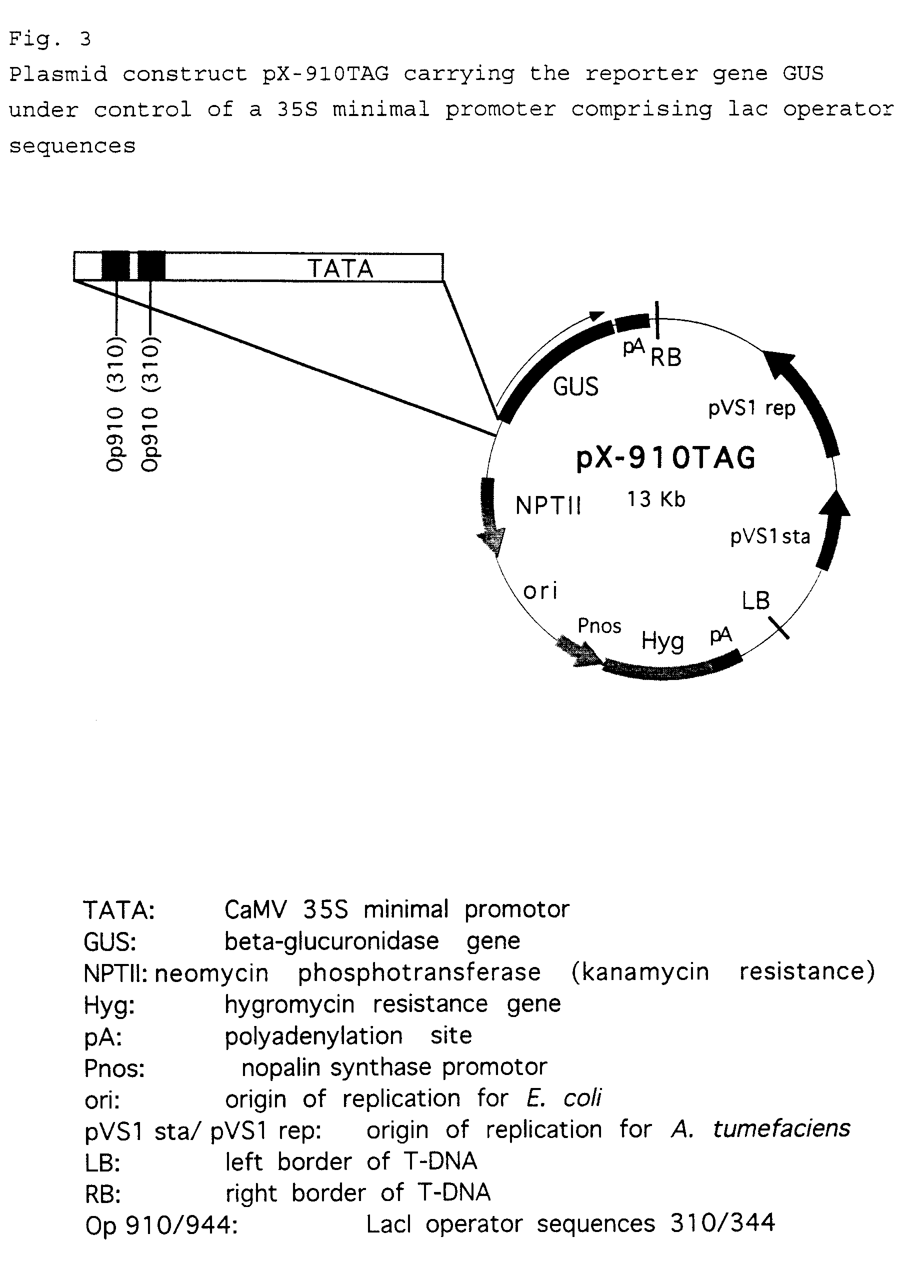

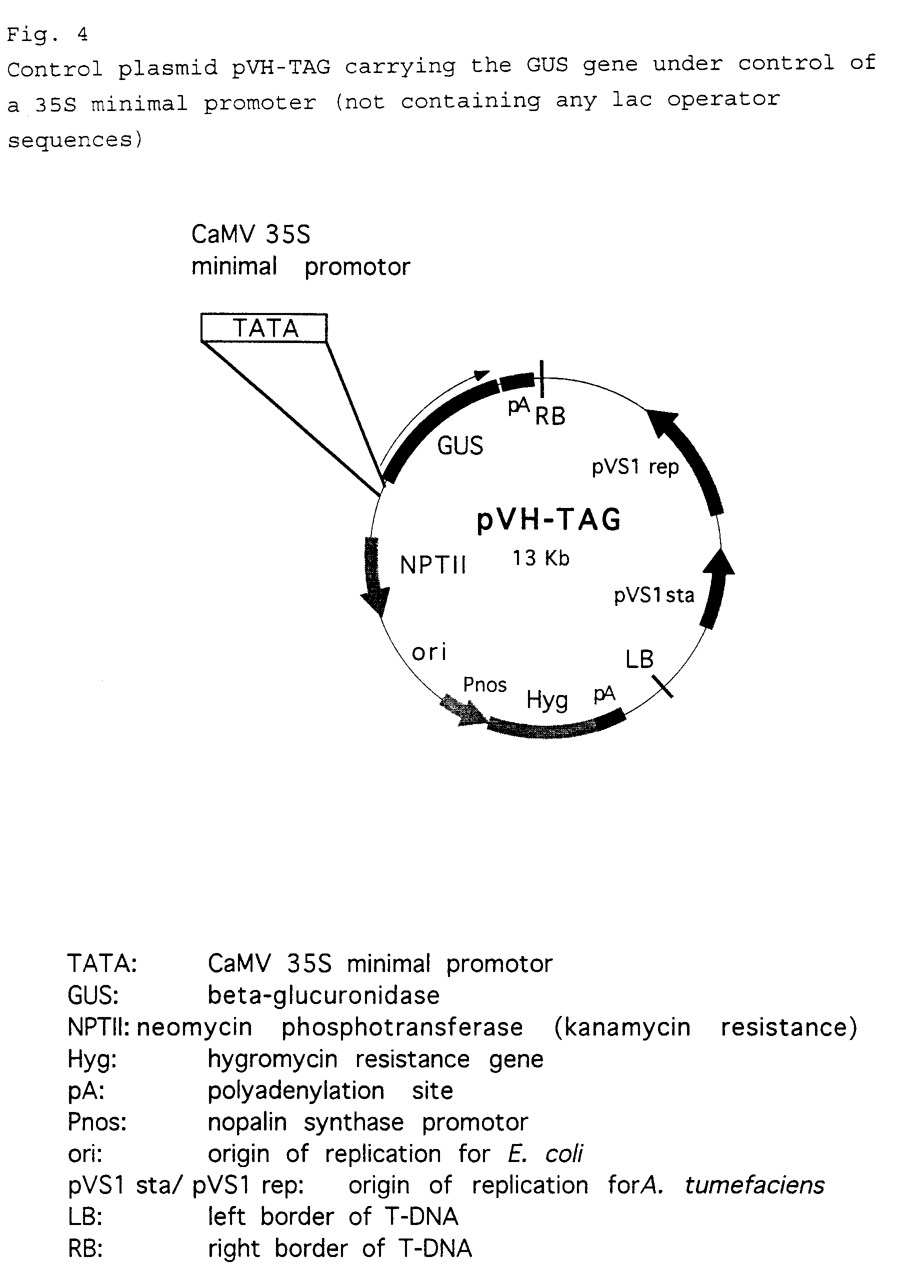

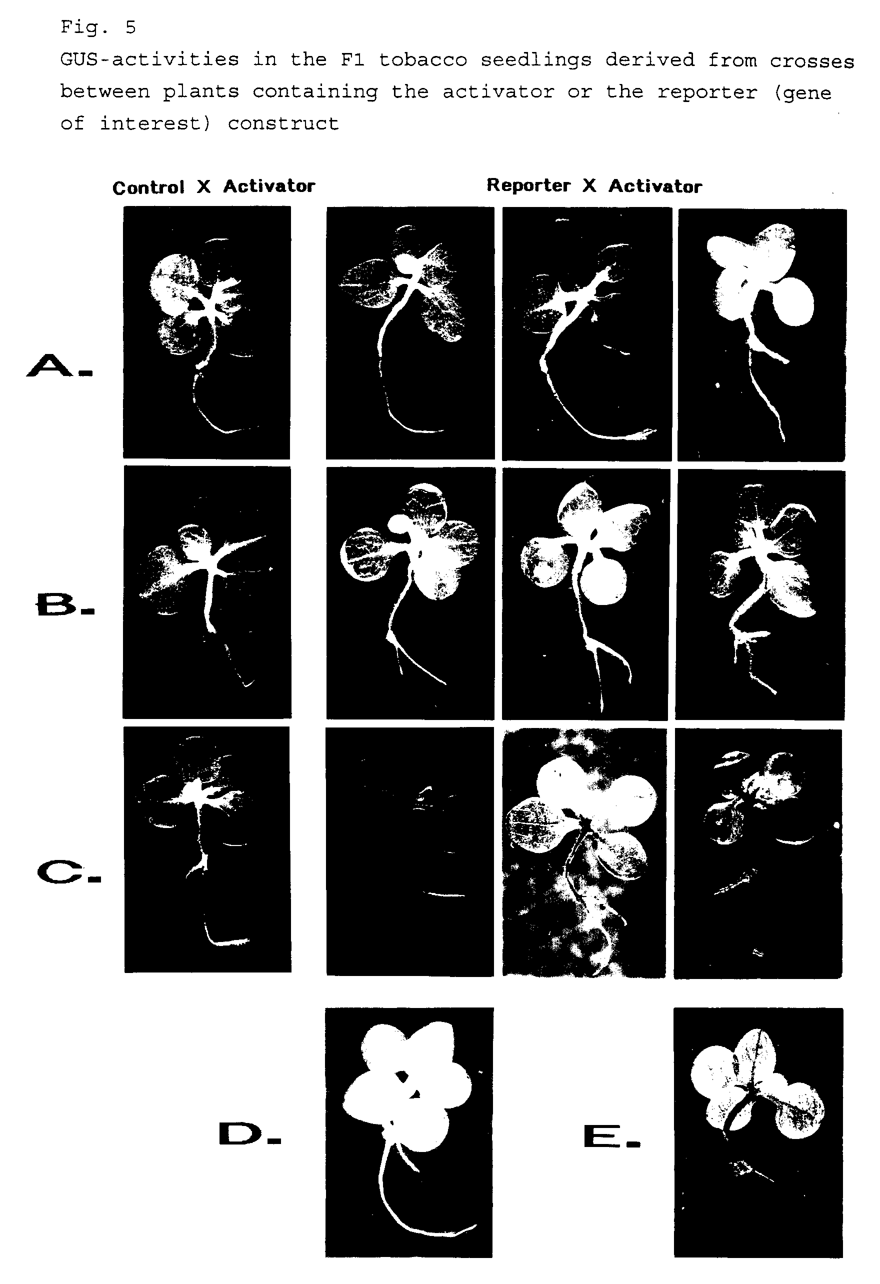

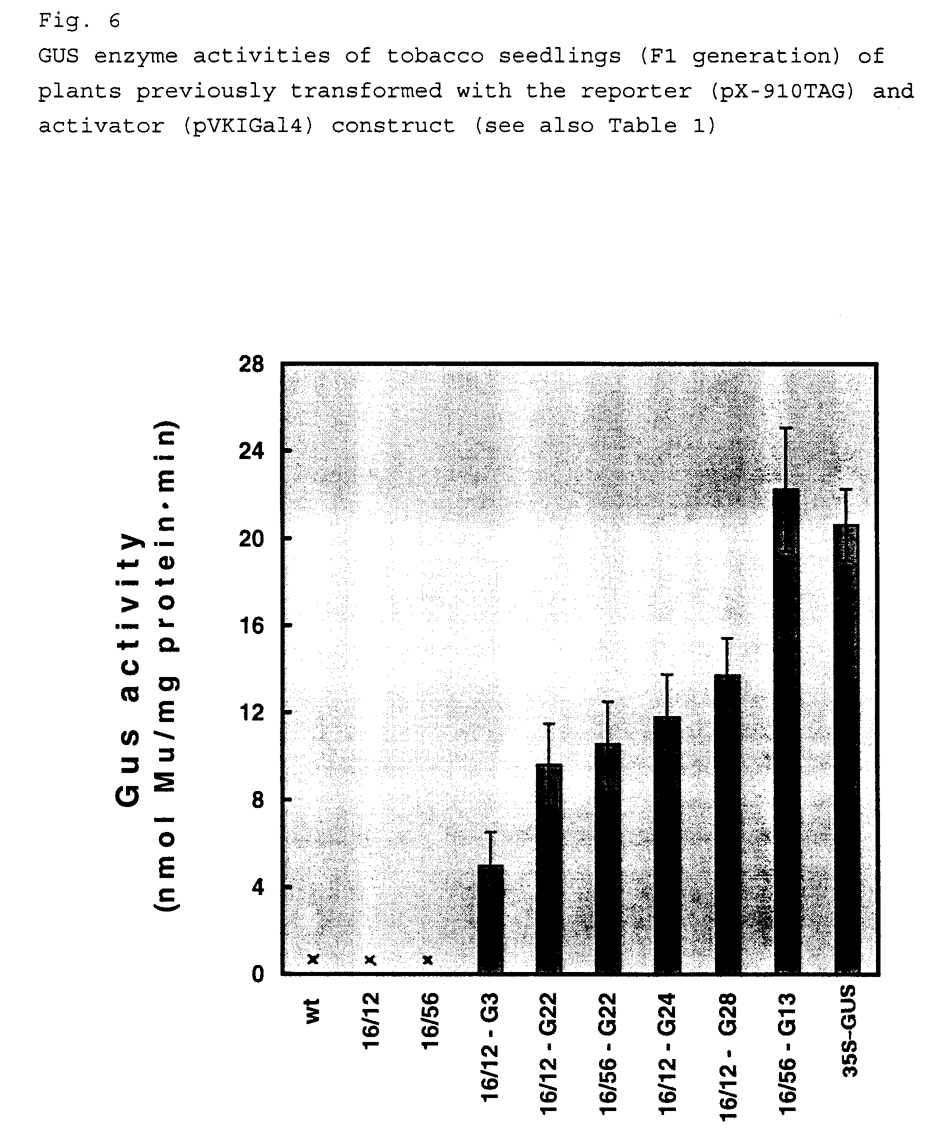

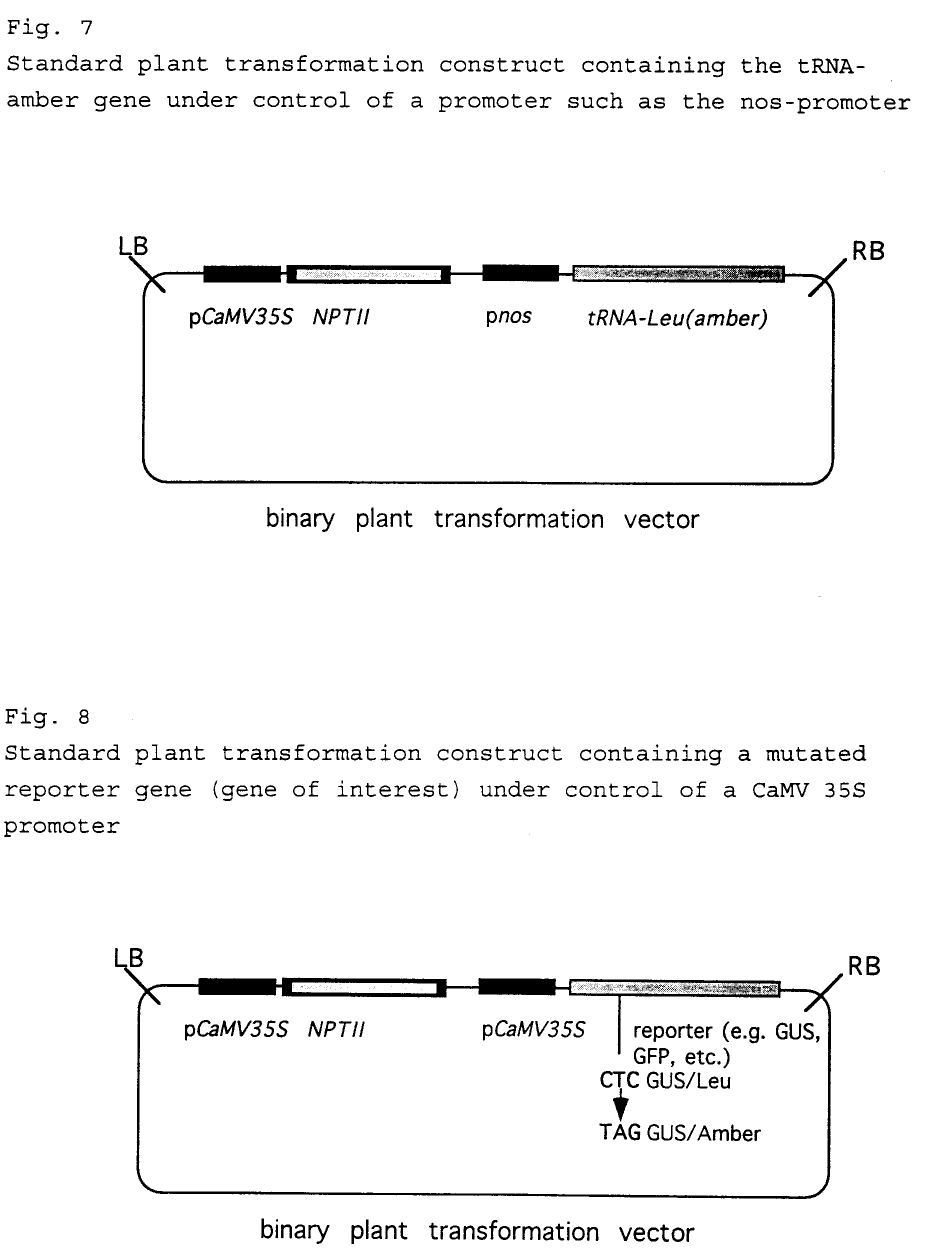

**Tab. 1**

| GUS enzyme activity of F1 tobacco seedlings of plants previously transformed with the activator and reporter construct (corresponding diagram in Fig. 6) | | | | | | | |
|---|---|---|---|---|---|---|---|
| | GUS Activity (nmol 4-MU · mg⁻¹ protein · min⁻¹) | | | | | | |
| | Assay 1 | Assay 2 | Assay 3 | Assay 4 | Assay 5 | MV | SD |
| wt | < 0.001 | < 0.001 | < 0.001 | < 0.001 | < 0.001 | | |
| 16/12 | < 0.001 | < 0.001 | < 0.001 | < 0.001 | < 0.001 | | |
| 16/56 | < 0.001 | < 0.001 | < 0.001 | < 0.001 | < 0.001 | | |
| 16/12-G3 | 3.22 | 3.96 | 7.25 | 5.45 | - | 4.97 | 1.54 |
| 16/12-G22 | 7.17 | 12.80 | 8.80 | 8.90 | 10.30 | 9.60 | 1.88 |
| 16/56-G22 | 9.60 | 13.80 | 10.10 | 8.70 | - | 10.55 | 1.94 |
| 16/12-G24 | 10.00 | 14.90 | 10.20 | 12.00 | - | 11.78 | 1.97 |
| 16/12-G28 | 14.90 | 11.00 | 3.55 | 15.40 | - | 13.70 | 1.71 |
| 16/56-G13 | 19.30 | 26.90 | 20.00 | 21.00 | 24.00 | 22.24 | 2.83 |
| 35S-GUS | 22.00 | 20.20 | 21.50 | 17.60 | 21.80 | 20.62 | 1.64 |
| Notes: MV = Mean Value, SD = Standard Deviation | | | | | | | |

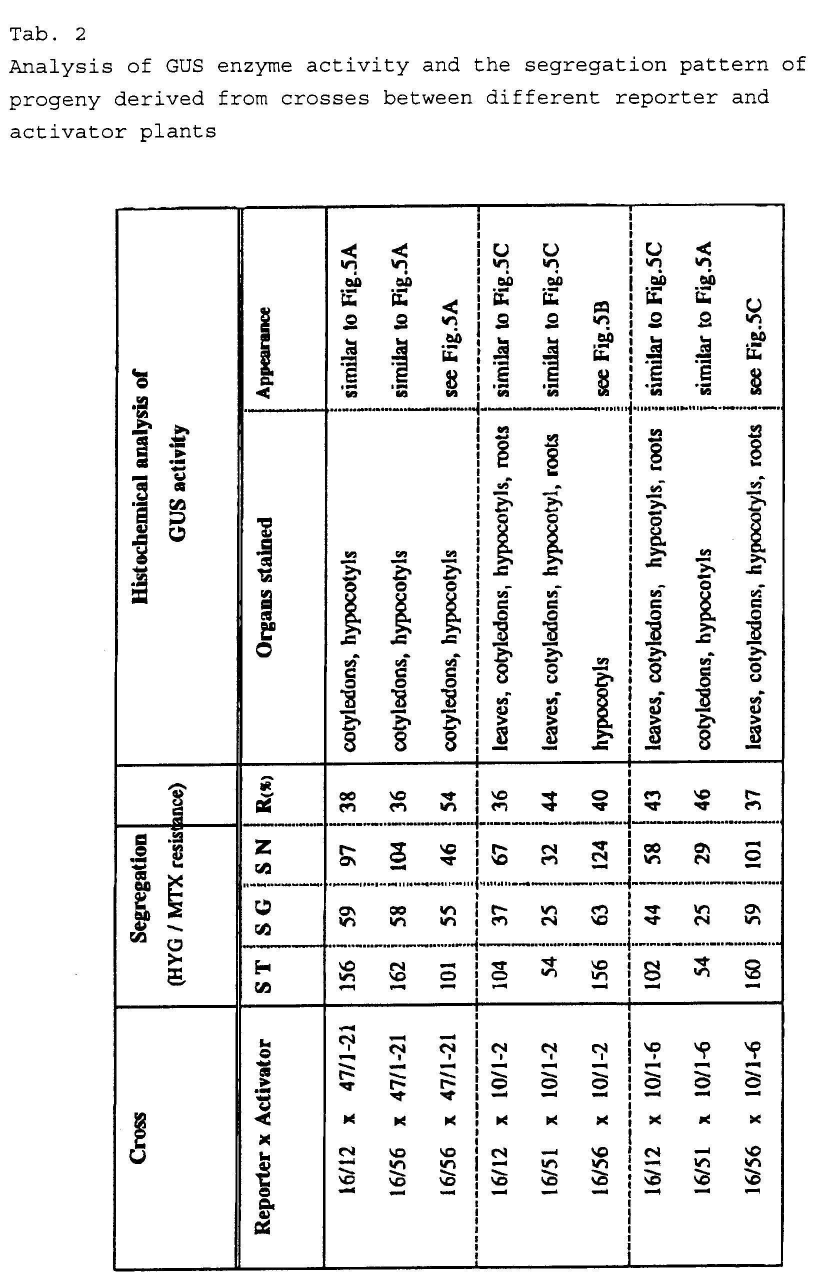

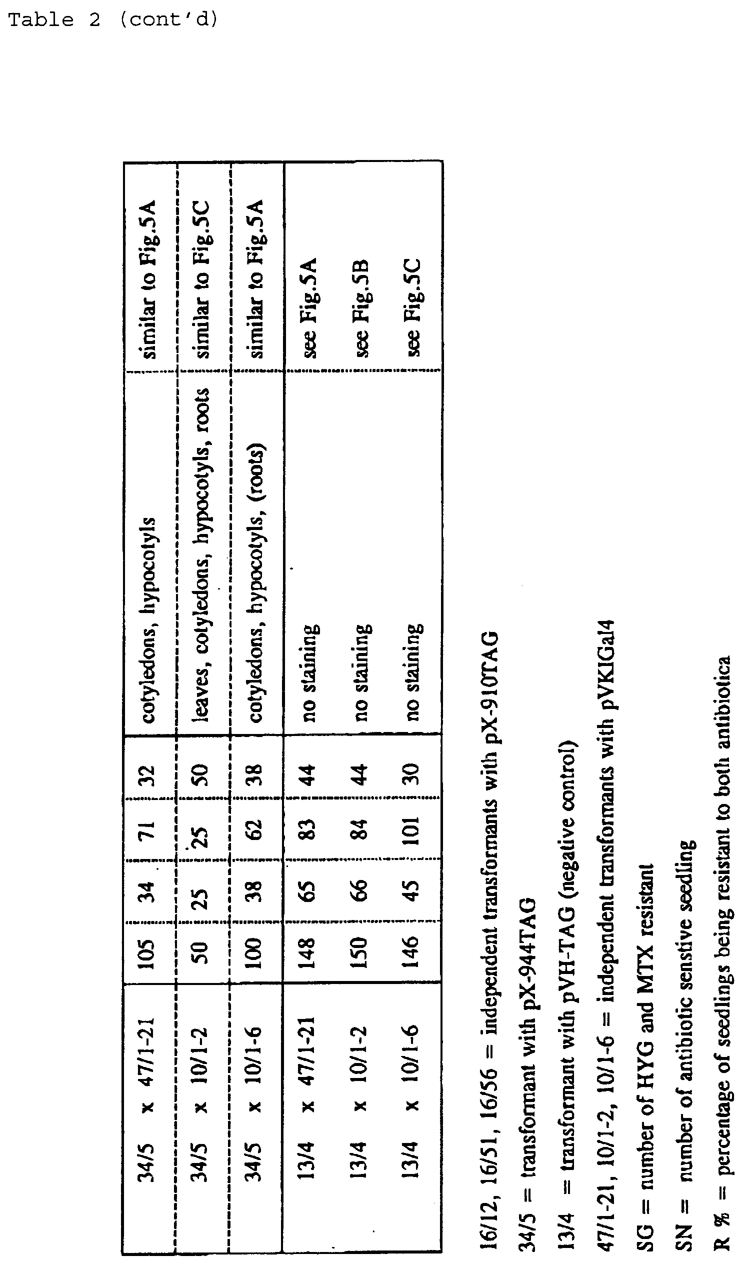

### DESCRIPTION OF THE FIGURES AND TABLES

Figure 1 shows the plasmid construct pVKIVGal4 carrying a LacI-Gal4 transcription activator fusion. In principle the LacI-Gal4 transcription activators were obtained by an in frame fusion of a tyrosine 17 -> histidine 17 substitutiion mutant (lacI^{his}) of the bacterial LacI gene and an transcription activating domain of the yeast GAL4 protein.
The coding sequence of the LacI DNA-binding mutant LacI^{his} was obtained from the Institute of Genetics at the University of Cologne. This mutant is described in detail in Lehming et al. (1987) Embo J. 6, 3145-3153. We got this LacI mutant in a vector (pWB100HIS1) derived from the pWB100 series, of which a plasmid map and some sequence data is given in Lehming et al., supra. In this semi-synthetic LacI^{his} gene the LacI translation initiation codon had been converted to ATG and incorporated into an NdeI site besides other introductions of restriction sites without changing the wild type protein sequence but with one exception: The LacI^{his} gene has an amino acid exchange at amino acid position 17 located in the operator recognition helix where the tyrosine is substituted by a histidine. To facilitate further cloning, a SpeI restriction site (5'-ACTAGT-3' encoding residues 329 and 330) was introduced into the C-terminus of the LacI^{his} gene converting residue 330 from leucine to serine. This amino acid change should have no influence on the desired function of the protein and was done only for convienience of plasmid construction.
The coding sequence of the transcription activation domain II (residues 768-881) of the GAL4 I+II protein was obtained from Dr. Jun Ma from the Harvard University of Cambridge, Massachusetts. The studies on the transcriptional activating segments of the GAL4 protein were presented by Jun Ma and Mark Ptashne, supra, where also the construction of the different GAL4 derivatives has been reported. The coding sequence of the GAL4 transcription activation domain II was inserted in frame into the unique above-mentioned SpeI restriction site of the LacI^{his} gene, generating the in frame LacI^{his}-Gal4 fusion gene that is used in the experiment described in EXAMPLE I. In this fusion gene the coding sequence for the transcription activating portion of GAL4, i.e. roughly domain II comprising amino acids 768-881, was inserted into the LacI coding region at the SpeI site indroduced at the position of codons 329 and 330 of the LacI coding region (see above). The construction of the fusion gene was done in the pWB100 derived vector carrying the modified LacI^{his}-gene like described above. In order to allow expression of the LacI^{his}-Gal4 fusion gene in plants, it was isolated as an NdeI-BglII restriction fragment using the NdeI restriction site at the modified lacI translation start site and the BglII site 3' of the lacI gene (cf pWB100 in Lehming et al., (1987) supra) and inserted into the AseI and BamHI restriction sites of the plant expression vector pKI102.
The plant expression vector pKI102 was made in house and contained a plant expression cassette consisting of a polylinker for the insertion of the coding sequence to be expressed between a Cauliflower Mosaic Virus 35S promotor and a polyadenylation signal. The 35S promoter consists of basepairs 7016 to 7434 of the CaMV strain Cabb B-D and the 35S polyadenylation signal corresponds to basepairs 7436 to 7639 of the CaMV strain Cabb B-D (CM 1841; Gardner et al. (1981) Nucl. Acids Res. 9, 2871-2888).
However, in principle any plant expression vector can be used. Suitable plant expression vectors, for instance binary plant transformation vectors, are meanwhile common tools frequently described in the literature and well-known to the person skilled in the art.
The expression and transcription activating ability of the LacI^{his}-Gal4 fusion was checked in tobacco protoplasts.
Having confirmed the functionality of the construct the whole expression cassette, consisting of the CaMV 35S promotor, the LacI^{his}-Gal4 fusion gene and the polyadenylation signal, was inserted as a PstI fragment into the binary vector pVK18 to generate the vector construction pVKIGal4. pVK18 corresponds to an usual plant transformation vector. pVK18 has a polylinker for inserting genes at its right border of the T-DNA and was derived from pMN016-VS, which is based on pMN001 (Reiss et al. (1994) Plant Phys. (Life Sci. Adv.) 13, 143-149) and which was modified in two aspects: (a) it contains a CaMV 35S promoter from the plasmid pDH51 (Pietrzak et al. (1986) Nucl. Acids Res. 14, 5857-5869) and (b) the mini-RK replicon of pMN001 was replaced by the mini-pVS replicon from pVS1 (Itoh et al. (1984) Plasmid 11, 206-220; Deblaere et al. (1987) Methods in Enzymology 153, 277-292). pVK18 was constructed from pMN016-VS by removing its pBR322 origin of replication, its beta-lactamase gene and the CaMV 35S-NPTII-polyA cassette and replacing them with the replication origin, the bacterial kanamycicin resistance marker and the polylinker of the plasmid pK19 (Pridmore (1987) Gene 56, 309-312).
pVKIGal4 is the final transcription activation construct that was used to generate stable transformants and is deposited in E. coli at the Deutsche Sammlung von Mikroorganismen und Zellkulturen GmbH (DSMZ), Braunschweig, Germany, under the deposition number DSM 11108.
In principle any plant transformation vector could be used to transform plants with the LacI^{his}-Gal4 fusion gene.

Figure 2 depicts the plasmid construct carrying the LacI-Vp16 fusion gene. In principle, the cloning strategy described in the description of Figure 1 for the LacI-Gal4 fusion gene was followed. From A.J. Levine at the Princeton University, New Jersey, we got a LacI-Vp16 fusion construct (pHCMVLAP348) that so far had only been tested in mammalian cells. The construct is described and its data are presented in Labow et al. (1990) Mol. Cell. Biol. 10, 3343-3356. The coding sequence of the LacI-Vp16 fusion was isolated from pHCMVLAP348 via BglII and a partial SacI digestion. In this fusion gene the coding sequence for the transcription activating portion of VP16, i.e. roughly the carboxyl-terminal acidic domain comprising amino acids 369-488, was inserted into the LacI coding region at about the codon for LacI amino acid 348 (Labow et al. (1990) supra). The isolated LacI-Vp16 fragment was inserted into the plant expression vector pKI102 downstream of the CaMV 35S promotor and upstream of the polyadenylation signal. pKI102 is a plant expression vector made in house (see description of Figure 1) but in principle any plant expression vector can be used to provide the LacI-Vp16 fusion coding sequence under control of plant expression signals.
In order to be able to generate stable transformed plants the complete CaMV 35S-LacI-Vp16 polyA expression cassette was isolated from the pKI102 derivative as a HindIII restriction fragment after partial digestion and inserted into the polylinker of the binary plasmid pVK18, generating the plasmid pVKIVp16.

Figure 3 shows the plasmid map of the plant expression vector pX-910TAG carrying the reporter gene GUS (the uidA gene from E. coli) under control of a CaMV 35S minimal promoter with lac operator sequences inserted upstream.
In order to monitor the activity of the LacI^{his}-Gal4 and LacI-Vp16 fusion constructs in plant cells, the plasmid pX-910TAG was constructed by the following procedure. The essential elements of the pX-910TAG and pX-944TAG constructs (pX-944TAG differing from pX-910TAG only in the lac operator sequence) are Lac operator sequences located upstream of a CaMV 35S minimal promoter (-46 to +8 relative to start of transcription [+1]) controlling the expression of a GUS gene.
In the presence of the chimeric LacI^{his}-Gal4 or LacI-VP16 transcription activator, the activator can bind to the cognate operator sequences and thus activate, facilitate and/or enhance the transcription and expression of a gene of interest, here the GUS reporter gene. It is worth noting that the T-DNA does not contain strong enhancer elements and the nearest promoter is more than 2 kb away, reducing the probability of the minimal promoter being activated in cis by neighbouring promoter elements. Similarly the gene of interest is situated between the minimal promoter and the T-DNA right border to minimize the chances of the minimal promoter being activated in cis by promoter elements in the flanking plant genomic DNA.
The expression of the GUS reporter gene in pX-910TAG and pX-944TAG can be easily monitored by measuring the GUS enzyme activity (e.g. according to the methods described by Jefferson et al. (1987) EMBO J. 6, 3901-3907 and Jefferson (1987) Plant Mol. Biol. Rep. 5, 387-405). In the absence of transcription activator there should be no detectable GUS expression above the background. In consequence the activity of the above described promoter is dependent on the presence of functional transcription activator protein, providing a very specific and stringent control mechanism of gene expression.
The two plasmids pX-910TAG and pX-944TAG differ only in the operator sequences inserted upstream of the CaMV 35S minimal promotor. The Lac operator sequences were obtained from the Institute of Genetics of the University of Cologne (plasmids pWB910 and pWB944) and are published under the designations 310 and 344, respectively, in Lehming et al. (1987), supra. These two operator sequences were chosen since it was found for bacteria that the wild-type Lac repressor and the His1 mutant Lac repressor described above are capable of binding tightly to the 310, and the 310 and 344 operator sequence, respectively (Lehming et al. (1987), supra; Lehming et al. (1989), supra).
The operator sequences were isolated as XbaI fragments from the plasmids pWB910 (310) and pWB944 (344) and first inserted into the unique XbaI site of the pBluescript vector (Stratagene, La Jolla, CA, USA). After cloning in pBluescript the operator sequences 310 and 344 were reisolated from the pBluescript derivatives using two flanking blunt end restriction sites of the pBluescript polylinker: Ecl136II and EcoRV. It was found out by restriction analysis that two XbaI operator fragments of 310 formed a double insertion into the XbaI restricted pBluescript vector. In consequence the corresponding Ecl136II-EcoRV fragment contained two 310 operators. The Ecl136II-EcoRV fragments containing either the operator sequences 310 or 344 were inserted 5' of a CaMV 35S minimal promoter-GUS-polyadenylation construction into the binary vector pVH-TAG. The blunt end Ecl136II-EcoRV fragments were inserted into the unique Ecl136II site of pVH-TAG, generating the plasmids pX-910TAG (carrying operator sequences 310 from plasmid pWB910) and pX-944TAG (carrying operator sequences 344 from plasmid pWB944). In the case of pX-944TAG a double insertion of the corresponding Ecl136II-EcoRV fragment took place as was learned from restriction analysis. In fact both reporter constructs, pX-910TAG and pX-944TAG, are containing two operator sequences (310 and 344, respectively).
As described above the reporter plasmids pX-910TAG and pX-944TAG were obtained by insertion of the operator sequences 310 (from plasmid pWB910) or 344 (from pWB944) into the binary vector pVH-TAG that was constructed in house (see also Fig. 4). In principle any plant transformation vector can be used. Here, the important elements of pVH-TAG are the GUS expression cassette comprising the GUS coding sequence under control of a CaMV 35S minimal promotor and the fact that after insertion of the operator sequences a regulatory unit dependent on LacI-derived transcription activators is obtained. In pVH-TAG, as a plasmid map of which is given in Figure 4, the GUS expression cassette consists of a GUS coding sequence with a polyadenylation signal and a CaMV minimal promoter. The GUS reporter gene (modified E. coli uidA coding sequence) was isolated together with the CaMV 35S polyadenylation signal from the plasmid pRT103GUS (Töpfer et al. (1988) Plant Cell Rep. 7, 225; Töpfer et al. (1993) Methods in Enzymology 217, 66-78). The CaMV 35S minimal promoter represents the minimal TATA region of the CaMV 35S RNA promoter and comprises the base pairs -46 to +8 around the transcription start ([+1]; as reference Odell et al. (1985) Nature 313, 810-812; Benfey and Chua (1990) Science 250, 959-966).
An Ecl136II (SacI) restriction site is located upstream of the CaMV 35S minimal TATA region of the GUS expression cassette in pVH-TAG. This unique restriction site was used to insert the operator sequences in the way described above, generating the plasmids pX-910TAG and pX-944TAG.
The lac operator sequences are as follows: wild type lac operator sequence (operator 1 in E. coli): AATTGTGAGCGGATAACAATT; lac operator consensus sequence: AATTGTGAGCSGCTCACAATT (S being G or C); 910 (ideal lac operator): AATTGTGAGC·GCTCACAATT; 944: AATTGTTAGC·GCTAACAATT (Lehming (1990) supra; Lehming et al. (1987), (1989) supra).
The GUS gene which was chosen because of its enzyme activity being easily measurable in plants, can in principle be replaced by any other reporter gene or gene of interest, that is desired to be under such transcription activator dependent gene expression control.
The plasmid pX-910TAG has been deposited in E. coli at the Deutsche Sammlung von Mikroorganismen und Zellkulturen (DSMZ), Braunschweig, Germany, under the deposition number DSM 11107.

Figure 4 depicts a plasmid map of the binary plant transformation vector pVH-TAG, described in detail above (description of Figure 3).

Figure 5 shows GUS enzyme activities in F1 tobacco seedlings. Tobacco plants containing the reporter construct pX-910TAG (Reporter, 16/56) and the control construct pVH-TAG (Control, 13/4) lacking the LacI-GAL4 binding sites served as female plants. These plants were pollinated with pollen from plants containing an activator construct: lines 47/1-21 (A), 10/1-2 (B) and 10/1-6 (C). From each crossing approximately 50 progeny seedlings (4 weeks old) were histochemically stained with X-Gluc. No GUS activity was detected in the progeny from crosses between activator and control plants, while the progeny of crosses between activator and reporter plants showed GUS activity. For each crossing three seedlings are shown representing typical GUS expression within the population of the progeny.
(D) No GUS activity was detectable in seedlings from plants transformed only with the reporter construct (Reporter, 16/56). (E) Constitutive expression of GUS activity in a tobacco seedling from a plant transformed with the positive control construct. This positive control 35S/GUS construct contains the GUS coding region under control of a fully active 35S RNA promoter, any plant transformation vector carrying the GUS reporter gene under control of a 35S promoter may be used as a positive control plasmid.

In Figure 6 GUS enzyme activities in different F1 tobacco seedlings are quantitively compared. Tobacco plants were transformed successfully with the activator and reporter construct. Seeds were harvested and germinated on MS medium containing hygromycin (15 mg/l) and methotrexate (0.2 mg/l).

After 4 weeks GUS-activity was determined in pools of 6 seedlings. Data represents means and standard deviation of 4 replicate samples. No activity was detectable in wild-type seedlings of SR1 tobacco and in seedlings 16/12 and 16/56 containing only the reporter construct.
SR1, 16/12 and 16/56 (negative controls), 16/12-G3, 16/12-G22, 16/56-G22, 16/12-G24, 16/12-G28, 16/56-G13 (double transformants), 35S-GUS (positive control, described above). The results shown in Figure 6 are also given in Table 1.

Figure 7 and Figure 8 show shematic plasmid maps of binary transformation vectors used for transformation of plants in order to activate expression of the GUS reporter gene by suppressor tRNAs in the progeny of the crossings between plants containing either the gene of interest (here, the GUS gene as example) containing a premature stop codon or a suppressor tRNA. In both vectors, the selectable marker NPTII is under control of the constitutive CaMV 35S promoter. RB, LB are the right and left borders of the T-DNA. These, and all other sequences refer to standard binary vectors described in the literature. Of course also binary vectors carrying other selectable marker genes can be used, e.g. in order to facilitate monitoring of the presence of two different DNA molecules (i.e. the construct encoding the suppressor tRNA encoding and construct encoding the gene of interest) in the same plant cell.

Tab. 1 shows the results of a fluorometric analysis of GUS enzyme activity in F1 tobacco seedlings of plants previously transformed with the activator and reporter construct. The results corresponds to those shown in Figure 6. Glucuronidase enzmye activity was quantitatively measured as described by Jefferson et al., supra. GUS enzyme activity is expressed in nmol Mu (4-methylumbelliferone) x mg⁻¹ protein x min⁻¹.

Tab. 2 shows the analysis of the GUS enzyme activity of the progeny derived from crosses between different reporter and activator plants as well as the segregation pattern of the seedlings in dependence on antibiotic restistance of the seedlings. Tobacco plants containing the reporter constructs pX-910TAG (lines: 16/12, 16/51, 16/56) or pX-944TAG (line: 34/5) or the control construct pVH-TAG (line 13/4 lacking the lac operator sequences) served as female plants. These transgenic plants are hygromycin (HYG) resistant due to the integration of the reporter or control construct containing the HYG resistance as selectable marker. These plants were pollinated with pollen from plants containing the activator construct pVKIGal4 (lines 47/1-21, 10/1-2 and 10/1-6) having a methotrexate (MTX) resistance as a selectable marker. From each crossing a given number of progeny seedlings (ST) were germinated on MS medium (Murashige and Skook (1973) Physiol. Plant 15, 473-497) containing the two antibiotics HYG and MTX (20 µg/ml plant medium hygromycin (Calbiochem, Frankfurt, Germany) and 0.2 µg/ml plant medium methotrexate (Sigma, Munich, Germany). The percentage (R) of the transgenic plants (SG) being resistant to both HYG and MTX was determined. From each crossing approximately 50 progeny seedlings (4 weeks old) were histochemically stained with X-Gluc. Seedlings resulting from the crossing of the control plant 13/4 (carrying the negative reporter control plasmid) and different activator plants show no GUS staining. The organs showing GUS activity are described in the Table and the appearance for the 16/56 crosses is shown in Figure 5.

### EXAMPLES

### EXAMPLE I

### Construction of the transgenic plant reporter lines

Nicotiana tabacum cv. Petit Havanna SR1 (Maliga et al. (1973) Nature New Biol. 244, 29-30) plants were transformed with the above-described reporter plasmids pX-910TAG (DSM 11107) or pX-944TAG by Agrobacterium tumefaciens-mediated transformation using the leaf disk technique described by Horsch et al. (1985) Science 277, 1229-1231. To infect tobacco leaf discs and to facilitate the reporter T-DNA transformation of the plants the agrobacterial strain A. tumefaciens pGV3101 pMP90 (Koncz and Schell (1986) Mol. Gen. Genet. 204, 383-396) was used. Several transgenic calli and finally plant lines were regenerated: lines 16/.. originating from the transformation experiments using pX-910TAG and lines 34/.. originating from transformation experiments using pX-944TAG. Transgenic plants were selected making use of the plant selectable marker for hygromycin resistance located on the T-DNA (see plasmid maps of pX-910TAG and pVH-TAG, Figures 3 and 4). 20 µg/ml plant medium hygromycin (Calbiochem, Frankfurt, Germany) were used during the selection process.
GUS assays were performed according to the protocols of Jefferson et al. (1987) supra, and no GUS activity could be detected in the reporter plants, as it was expected in the absence of the corresponding transcription activators. To monitor the induction of GUS activity, some plant reporter lines were selected and retransformed with activator constructs and/or crossed to several previously regenerated activator plants (as described in the following Examples).

### EXAMPLE II

### Construction of the transgenic plant activator lines

Nicotiana tabacum cv. Petit Havanna SR1 (Maliga et al. (1973) Nature New Biol. 244, 29-30) plants were transformed with the above-described activator plasmids pVKIGal4 (DSM 11108) or pVKIVp16 by Agrobacterium tumefaciens-mediated transformation using the leaf disk technique described by Horsch et al. (1985) Science 277, 1229-1231. To infect tobacco leaf discs and to facilitate the activator T-DNA transformation of the plants the agrobacterial strain A. tumefaciens pGV3101 pMP90 (Koncz and Schell (1986) Mol. Gen. Genet. 204, 383-396) was used. Several transgenic calli and finally plant lines were regenerated: lines 10/.. and 47/.. originating from transformation experiments using pVKIGal4 and other lines (not mentioned in the Figure Descriptions) originating from transformation experiments with pVKIVp16. Transgenic plants were selected making use of the plant selectable marker for methotrexate resistance (dihydrofolatereductase, DHFR) located on the T-DNA (see plasmid maps of pVKIGal4 and pVKIVp16, Figures 1 and 2). 0.2 µg/ml plant medium methotrexate (Sigma, Munich, Germany) were used during the selection process.
The transgenic activator plants showed wild type phenotype without any observed abnormalities and were cultivated in the greenhouse. Several activator plants were randomly selected and crossed to reporter lines (see EXAMPLE I).

### EXAMPLE III

### Retransformation of transgenic reporter lines with activator constructs.

To shorten the process (plant cultivation, crossing, seed harvesting etc.) and get some data on the functionality of the transactivation system of the invention, reporter plants (see EXAMPLE I) were retransformed with the above-described activator plasmids pVKIGal4 or pVKIVp16 using the leaf disk transformation technique described by Horsch et al. (1985) Science 277, 1229-1231. To infect leaf discs of the transgenic reporter tobacco lines and to facilitate the activator T-DNA transformation of the plants the agrobacterial strain A. tumefaciens pGV3101 pMP90 (Koncz and Schell (1986) Mol. Gen. Genet. 204, 383-396) was used. Several double transgenic calli and finally plant lines were regenerated. Some of them are mentioned in Figure 6, e.g. 16/12-G3 and 16/56-G22. The retransformed plants were selected making use of the plant selectable marker for methotrexate resistance located on the T-DNA of the activator plasmids pVKIGal4 and pVKIVp16. 0.2 µg/ml plant medium methotrexate were used during the selection process.
X-Gluc staining and quantitative GUS assays, i.e. histochemical and fluorometric analyses of β-glucuronidase enzmye activity were performed as described by Jefferson et al. (1987) EMBO J. 6, 3901-3907 and Jefferson (1987) Plant Mol. Biol. Rep. 5, 387-405. et al.. The retransformed calli, the regenerated primary double transformants and the F1 generation of the double transformants were analyzed and some of the results are shown in Figure 6 and Table 1. GUS enzyme activity was expressed in nmol Mu (4-methylumbelifferone) x mg⁻¹ protein x min⁻¹; X-Gluc (5-bromo-4-chloro-3-indolyl-β-D-glucuronic acid) is available from Sigma, Munich, Germany.
It was found that expression of the reporter gene GUS was efficiently transactivated in the presence of LacI derived transcription activators in the double transgenic plant lines (see Figure 6 and Table 1). Surprisingly, GUS expression levels were even as high as those of the 35S promoter known as a very strong constitutive promoter (positive control line). In the transgenic tobacco line 16/56-G13 the detected GUS activity was even higher than in the control line.
It is worth mentioning that similar results were obtained when the activator and reporter plasmids were transiently expressed in tobacco protoplasts. Thus the transactivation system of the invention may also be successfully employed when transient expression in plant cells is desired.

### EXAMPLE IV

### Crossing trasngenic reporter lines with transgenic activator lines

Several activator plants (see EXAMPLE II) were crossed with several reporter lines (see EXAMPLE I). The seeds of the crosses were germinated on MS medium (Murashige and Skoog (1973) Physiol. Plant 15, 473-497) containing 20 µg/ml plant medium hygromycin (Calbiochem, Frankfurt, Germany) and 0.2 µg/ml plant medium methotrexate (Sigma, Munich, Germany) to select the F1 progeny that is transgenic for both, reporter and activator constructs.
X-Gluc staining and quantitative GUS assays performed according to the protocols of Jefferson et al. (1987) supra, of the hygromycin and methotrexate resistant F1 generation demonstrate the induction of GUS activity in the presence of LacI derived transcription activators. The results are given in Figure 5 and Table 2. Plants transformed with a CaMV 35S-GUS construct (positive control construct) were used as a positive control for the GUS assays and as a comparative standard for the induction level.

### EXAMPLE V

### The suppressor tRNA transactivation system in plants

tRNA genes (e.g. tRNA Leu, tRNA Ser, etc.) from Saccharomyces cerevisae have been used. The genome of S. cerevisiae is fully sequenced and DNA sequences are available from public databases (e.g. NCBI). For example, the sequences of the mentioned tRNA genes can be obtained under the following accession numbers: K01869 for the yeast tRNA-Leu-3 gene; X74268 for the yeast tRNA-Ser gene; and X06992 for the yeast minor tRNA-Ser (AGY) gene. However any other tRNA gene suitable for expression in plants may be successfully employed for this transactivation system of the invention. Isolation and cloning of tRNA-amber genes is also described in Carneiro et al. (1993) Plant Mol. Biol. 22, 681-690.
Respective tRNA genes were site-specifically mutated to encode each of the three anticodon sequences (CUA, UUA, and UCA) that recognize, respectively, the amber, ochre and opal stop codons. The genes were subcloned into common pBluescript vector (Stratagene, La Jolla, CA, USA). Site directed mutagenesis was performed using published standard methods, such as Kunkel et al. (1985) Proc. Natl. Acad. Sci. USA 82, 488-492; Kunkel et al. (1985) Meth. Enzymol. 154, 367-382. The genes were transfered into the plant transformation vector pVK18 (see above description of the plasmid constructions). The resulting plasmid in which the suppressor tRNA gene (e.g. the amber tRNA^{Leu} gene) is under control of, for instance, the nos-promoter is schematically represented in Figure 7.
A GUS reporter gene such as published by Pouteau et al. (1991) was inactivated by introducing through site-directed mutagenesis (Kunkel et al., supra) stop codons into Leu codons, such as in position of amino acid 32 (Carneiro et al. (1993) Plant Mol. Biol. 22, 681-690). The mutant GUS gene was introduced into a plant transformation vector such as pVK18 under control of a rather strong promoter, such as e.g. the 35S RNA promoter.
DNA cloning, manipulation and analyses were all performed according to standard methods (e.g. Sambrook et al., supra). Of course any suitable binary vector can be used for the transformation of plants with the construct encoding the suppressor tRNA and the construct encoding the gene of interest comprising at least one premature stop codon, preferred an amber stop codon. For convenience during the transformation, selection and regeneration procedures the construct should contain different selectable markers.
The transgenic plants carrying the gene of interest (reporter plants) and the plants carrying the suppressor tRNA (activator plants) were treated in the same manner as described in EXAMPLES III and IV, i.e. F1 generations derived from crosses between reporter and activator (suppressor tRNA) plants were analyzed for GUS enzyme activity. Similar results to those shown in Figures 5 and 6, and Tables 1 and 2 were obtained upon co-expression of the constructs (while no GUS activity was detected in plants only containg the GUS gene construct).

## Claims

1. A chimeric transactivator protein active in plants, comprising (i) a functional portion of a DNA binding protein and (ii) a functional portion of a transcriptional activator protein.

2. The chimeric transactivator protein of claim 1 in which the DNA binding protein is the bacterial lacI protein, preferably a mutant of the lacI protein having at least one amino acid exchange, more preferably a mutant of the lacI protein in which the DNA recognition helix of the lacI protein has an amino acid exchange at position 1, most preferably a mutant of the lacI protein in which the wild type amino acid tyrosine (17) in position 1 of the DNA recognition helix is exchanged for histidine.

3. The chimeric transactivator protein of claim 1 or 2 in which the the transcriptional activator protein is GAL4 of Saccharomyces cerevisiae or VP16 of Herpes Simplex Virus (HSV).

4. The chimeric transactivator protein of claim 1 in which the transcriptional activator is GAL4 of S. cerevisiae or VP16 of HSV and the DNA binding protein is a mutant of the lacI protein having at least one amino acid exchange, preferably a mutant of the lacI protein in which the DNA recognition helix of the lacI protein has an amino acid exchange at position 1, and more preferably a mutant of the lacI protein in which the wild type amino acid tyrosine (17) in position 1 of the DNA recognition helix is exchanged for histidine.

5. The chimeric transactivator protein of claim 4 in which a portion of GAL4, from about amino acid 768 to amino acid 881, is inserted into the lacI or lacI mutant coding sequence at about the codon for lacI amino acid 330 or in which a portion of VP16, from about amino acid 369 to amino acid 488, is inserted into the lacI or lacI mutant coding sequence at about the codon for lacI amino acid 348.

6. The chimeric transactivator protein encoded by the plasmid pVKIGal4 (DSM 11108).

7. A recombinant nucleic acid molecule coding for the chimeric transactivator protein of any one of claims 1 to 5.

8. Plasmid pVKIGal4 (DSM 11108)

9. Plasmid pX-910TAG (DSM 11107).

10. A recombinant nucleic acid molecule coding for a suppressor tRNA which provides expression of a gene of interest containing a premature stop codon in plants, preferably coding for a suppressor tRNA from Saccharomyces cerevisiae, and more preferably coding for an amber suppressor tRNA.

11. A transgenic plant comprising a transgene coding for the chimeric transactivator protein of any one of claims 1 to 5.

12. A transgenic plant comprising a recombinant nucleic acid molecule of any one of claims 7 to 10.

13. A trangenic plant comprising plasmid pVKIGal4 (DSM 11108) and/or plasmid pX-910TAG (DSM 11107).

14. Parts and products of a plant of any one of claims 11 to 13, including protoplasts, plant cells, calli, seeds, tubers, cuttings, etc., as well as the progeny of these plants.

15. A method of controlling the expression of a gene of interest in a transgenic plant comprising utilising a chimeric transactivator protein active in plants according to any one of claims 1 to 6 to specifically regulate the expression of the gene of interest, which comprises at least one chimeric transactivator protein-responsive element.

16. A method of controlling the expression of a gene of interest in plant cells comprising utilising a chimeric transactivator protein active in plants according to any one of claims 1 to 6 to specifically regulate the expression of the gene of interest, which comprises at least one chimeric transactivator protein-responsive element, wherein the chimeric transactivator protein and the gene of interest are transiently expressed.

17. The method of claim 15 or 16 in which the chimeric transactivator protein-responsive element is the wild type Lac operator sequence AATTGTGAGCGGATAACAATT (lac operator 1) or the lac operator consensus sequence AATTGTGAGCSGCTCACAATT (S being G or C), preferably in which the chimeric transactivator protein-responsive element differs from the wild type Lac operator sequence or the lac operator consensus sequence in one or more base-pairs and/or in which the chimeric transactivator protein-responsive element has lost one or more base-pairs in comparison to the wild type Lac operator sequence or the lac operator consensus sequence, more preferably in which the chimeric transactivator protein-responsive element is the idealised Lac operator sequence AATTGTGAGC·GCTCACAATT or a modified Lac operator having the sequence AATTGTTAGC·GCTAACAATT.

18. The method of claim 15 or 16 in which the plasmid pX-910TAG (DSM 11107) and the plasmid pVKIGal4 (DSM 11108) are transferred into plants.

19. A method of controlling the expression of a gene of interest in a transgenic plant comprising utilising a suppressor tRNA active in plants, preferably a suppressor tRNA from yeast, and more preferably an amber suppressor tRNA, to specifically regulate the expression of a gene of interest, which comprises at least one premature stop codon, preferably a premature amber stop codon.

20. A method of controlling the expression of a gene of interest in plant cells comprising utilising a suppressor tRNA active in plants, preferably a suppressor tRNA from yeast, and more preferably an amber suppressor tRNA, to specifically regulate the expression of the gene of interest, which comprises at least one premature stop codon, preferably a premature amber stop codon, wherein the suppressor tRNA and the gene of interest are transiently expressed.

21. A method for producing plants having a desired phenotypic trait, the method comprising
- transforming a first plant with a recombinant nucleic acid molecule coding for a chimeric transactivator protein according to any one of claim 1 to 6,
- transforming a second plant with a recombinant nucleic acid molecule coding for a gene of interest which comprises at least one lacI (or lacI mutant)/GAL4-transactivator or lacI (or lacI mutant)/V16-responsive element,
- crossing of first transgenic plant with second transgenic plant and
- resulting in transactivation of expression of a gene of interest.

22. The method of claim 21 wherein one plant is transformed with plasmid pX-910TAG (DSM 11107) and the other plant is transformed with pVKIGal4 (DSM 11108).

23. A method for producing plants having a desired phenotypic trait, the method comprising
- transforming a first plant with a recombinant nucleic acid molecule encoding a suppressor tRNA, preferably a suppressor tRNA of yeast and more preferably an amber suppressor tRNA,
- transforming a second plant with a recombinant nucleic acid molecule encoding a gene of interest carrying a premature stop codon, preferably a premature amber stop codon,
- crossing of first transgenic plant with second transgenic plant and
- resulting in transactivation of expression of a gene of interest.

24. The method of any one of claims 15 to 23 in which the product of the gene of interest is detrimental to the plant cell.

25. The method of any one of claims 15 to 24 in which the phenotypic trait is production of a cytotoxic product in plant cells.

26. The method of any one of claims 15 to 25 in which the phenotypic trait is cell death.
